# EUROPEAN PATENT SPECIFICATION

(11) **EP 4 302 787 B1**
(45) Date of publication and mention of the grant of the patent: **27.08.2025**
(21) Application number: 22183895.6
(22) Date of filing: 08.07.2022
(51) Int. Cl.: A61L 26/00, A61L 27/36, A61L 27/52, A61L 27/54, A01N 63/40, A61K 35/76

(54) **HYDROXYETHYCELLULOSE GEL COMPOSITIONS COMPRISING BACTERIOPHAGES**
HYDROXYETHYCELLULOSEGELZUSAMMENSETZUNGEN MIT BAKTERIOPHAGEN
COMPOSITIONS DE GEL HYDROXYÉTHYCELLULOSE COMPRENANT DES BACTÉRIOPHAGES

(43) Date of publication of application: 10.01.2024
(73) Proprietor: PHATEC GMBH, 22301 Hamburg (DE)
(72) Inventor: Junghans, Simon Frank, 22301 Hamburg (DE)
(74) Representative: Simmons & Simmons LLP (Munich)

(56) References cited:
- WO-A1-2022/133532
- US-A1- 2010 291 041
- CARBOL JASON ET AL: "Formulating Topical Products Containing Live Microorganisms as the Active Ingredient", PHARMACEUTICAL TECHNOLOGY, vol. 42, no. 3, 1 March 2018 (2018-03-01), pages 32 - 36, XP093008434
- CHANG RACHEL YOON KYUNG ET AL: "Hydrogel formulations containing non-ionic polymers for topical delivery of bacteriophages", INTERNATIONAL JOURNAL OF PHARMACEUTICS, ELSEVIER, NL, vol. 605, 30 June 2021 (2021-06-30), XP086713571, ISSN: 0378-5173, [retrieved on 20210630], DOI: 10.1016/J.IJPHARM.2021.120850

## Description

The present invention relates to hydrogels comprising hydroxyethyl cellulose (HEC), bacteriophages, CaCl₂ and glycerol, and to uses thereof, especially in the treatment and prevention of bacterial infections. The present invention is further defined in the appended claims.

Bacterial infections of vascular grafts represent a major burden in cardiovascular medicine, which is related to an increase of morbidity and mortality. Different factors that are associated to this medical field like patient frailty, biofilm formation or immunosuppression negatively influence antibiotic treatment, and, thereby, inhibit therapeutic success. Thus, further treatment strategies are required. Bacteriophage antibacterial properties were discovered 100 years ago, but the focus on antibiotics in Western medicine since the mid-twentieth century slowed the further development of bacteriophage therapy. Therefore, the experience and knowledge gained until then in bacteriophage mechanisms of action, handling, clinical uses and limitations were largely lost. However, the parallel emergence of antimicrobial resistance and individualized medicine result in the need of further means for the treatment of bacterial infections.

This especially applies to the treatment of bacterial infections in the context of surgery. In particular, both open and endovascular surgical cardiovascular repair carry the risk of nosocomial infection and a prosthetic vascular graft infection is generally tedious, related to other complications, and often acutely life-threatening. The prevalence of nosocomial infections in surgical patients ranges from 4% to 15% in patients requiring intensive care treatment. The standard treatment of resection and autologous vascular reconstruction can be prohibitively high-risk in compromised patients and alternatives are urgently required. In addition, prosthetic vascular graft infection is one of the most feared complications in cardiovascular surgery and associated with significant morbidity, mortality as well as increased hospitalization costs. The most common pathogenic bacteria are *Staphylococcus aureus, Staphylococcus epidermidis,* other coagulase-negative staphylococci, enterobacterales, *Escherichia coli, Pseudomonas,* and corynebacteria. Bacteria often increase their virulence by attaching themselves to prosthetic material, protecting themselves from the local immune response and from antibiotics with the help of biofilms that inhibit phagocytosis and the current standard therapy. Moreover systemic antibiotic therapy is often inadequate because effective saturation concentrations can rarely be achieved in inflammatory periprosthetic tissue.

Bacteriophages (or simply 'phages'; from the Greek: "bacteria eater") are viruses that selectively infect bacterial cells and were first described in 1917 by the Canadian Félix Hubert d'Hérelle. They are quite stable in the environment and contribute significantly to the regulation of global bacterial mass. In principle, a bacteriophage can be found wherever its corresponding bacterium is but can only multiply where the host is. Bacteriophages are specific and almost always only affect strains within one bacterial species, rarely crossing species boundaries. Lytic bacteriophages initiate the lytic cycle of viral reproduction; phages kill their corresponding bacteria through lysis: once infected, the bacterium host cell then starts the process of reproduction, the destruction of the bacterium, and the release of new phage particles; this process is controlled by enzymes and an interaction of bacterial and phage genes. On the other hand, lysogenic phages initiate the lysogenic cycle: the bacteriophage nucleic acid is integrated into the host bacterium's genome or forms a circular replicon in the bacterial cytoplasm. Here the lytic circle is started by extern triggers, for example by UV-Light or warmth.

Bacteriophages are dynamic biological agents that multiply in the host bacteria and place different demands on clinical studies and complicate regulatory approval processes more suited to passive, chemical defined drugs like antibiotics. Although the therapeutically use of bacteriophages is common in the countries of the former Soviet bloc, there is a lack of randomized, placebo-controlled and double-blind studies that would provide scientifically usable data to allow regulatory approval in the Western hemisphere. An increasing number of case reports describe successful phage therapy to treat life-threatening infections. However, although some clinical studies have been undertaken, most failed to provide clear evidence of the effectiveness of phage therapy: one study assessing a phage cocktail against *E. coli* and *P. aeruginosa* infections in burn wounds was terminated early due to insufficient effectiveness.

Only bacteriophage solutions are currently available so topical application is difficult due to the adhesive and flow dynamics. Even if bacteriophages are successfully delivered to the site of infection, they can run into and onto the surrounding tissue, particularly in the case of circular application, such as infected anastomoses. Although this does not normally present a risk of local or systemic adverse reactions, it will compromise efficacy. Preservation of bacteriophages on prosthetic surfaces, for instance, is not known. It is essential that the bacteriophages remain in / at the place of application for sufficient time to ensure bacteriophage and bacterium interaction; semi-solid and solid formulations are needed.

Carbol et al. (Pharm. Technol., 42(3), 32-36 (2018)) describe bacteriophage containing hydrogels for topical applications.

Chang et al. (Int. J. Pharmac., 605, 12850 (2021)) and WO 2022/133532 describe bacteriophage containing hydrogels for the treatment of wound infections.

In view of the above, it remains an object in the art to provide antibacterial compositions, which can for example be applied during surgery.

The present invention provides a hydrogel comprising hydroxyethyl cellulose (HEC), bacteriophages, CaCl₂ and glycerol.

In the context of the present invention (see Example 1), the inventor has found that HEC is a gel forming agent which is especially suitable for preparing gels with the desired haptic and other properties. Especially, the hydrogel of the present invention can be steam sterilized, which is an important property for the envisaged use in the treatment of bacterial infections, especially during surgery.

In addition, as shown in Example 2, it could be shown that the claimed composition is particularly effective in treating bacterial infections when applied during surgery.

In the context of the present invention, the terms "phage(s)" and "bacteriophage(s)" are used interchangeably.

In the context of the present invention, the following abbreviations are used:

| | |
|---|---|
| AMR | Antimicrobial resistance |
| CT | Computed tomography |
| EVAR | Endovascular aortic repair |
| MRSA | Methicillin-resistant *Staphylococcus aureus* |
| PET | Positron emission tomography |
| SAVR | Surgical aortic valve replacement |
| TAVR | Transcatheter aortic valve replacement |
| TEVAR | Thoracic endovascular aortic repair |

As discussed above, bacteriophages are well-known anti-bacterial agents. In the context of the present invention, any type of bacteriophages can be used. It is also possible to prepare customized gel preparations where the type of bacteria to be treated is determined and then the corresponding bacteriophages are selected.

In another embodiment of the present invention, a standardized bacteriophage composition is used which are active against preselected types of bacteria. For example, the bacteriophages contained in the hydrogel of the present invention may be active against *Staphylococcus aureus, Staphylococcus epidermidis,* other coagulase-negative staphylococci, enterobacterales, *Escherichia coli, Pseudomonas,* and corynebacteria. Such bacteriophages are known in the art, as discussed above.

In the context of the present invention, it is preferred to use lytic bacteriophages. This has the advantage that that the antibacterial effect of the bacteriophages starts immediately when encountering a corresponding bacterium. In another preferred embodiment, the bacteriophages in the hydrogel of the present invention are thermostable.

The concentration of the phages in the hydrogel can be any concentration, which is effective for the treatment of the bacterial infection and may vary depending on the bacteriophages used and on the expected bacterial concentration.

As a minimum, a phage concentration of 10² pfu / ml may for example be used.

In one embodiment, the concentration of the bacteriophages is between 10² pfu/ml and 10⁸ pfu/ml, preferably between 10⁴ pfu/ml to 10⁸ pfu/ml or 10⁸ pfu/ml to 10⁷ pfu/ml.

Before adding the phages to the composition of the invention, the bacteriophages preferably are sterilized, more preferably steam sterilized. The initial phage concentration is may reduced accordingly during the sterilization process. This reduction of the phage quantity is calculated beforehand, so that the final product may have a phage concentration of at least 10² pfu / ml.

In another preferred embodiment, the bacteriophages are sterilized via sterile filtration.

The bacteriophages may be added in a solution to the hydrogel of the present invention. For example, when preparing the hydrogel of the present invention, the bacteriophage solution may be 50 %, and the HEC gel stock solution (optionally with other ingredients, see below) may also be 50 %.

The bacteriophage preparation may be produced according to the applicable GMP guidelines.

The hydrogel of the present invention is a hydoxy-ethly-cellulose (HEC) containing hydrogel.

Hydroxy-ethyl-cellulose (= HEC) has the following chemical formula 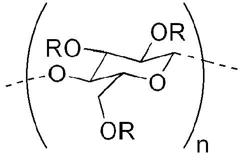
R = H or CH₂CH₂OH
(source: https://apothekenwiki.com/wp-content/uploads/2017/12/Hydroxyethylcellulose-Strukturformel.png)

HEC is known in the art. In the context of the present invention, HEC of any suitable length can be used. Especially, n can be varied between 300 and 15.000, more preferable between 500 and 10.000 most preferred between 2.000 and 8.000.

In the context of the present invention, HEC is used as a gel former because it could be shown in the context of the present invention that HEC leads to a stable gel scaffold on an aqueous basis. The gel matrix formed by the HEC offers sufficient space to retain the defined concentration of phage in a distribution that is conducive to release. In addition, the viscosity of the end product is easily adjustable via the concentration of the gel former, so that the change in the HEC percentage offers further product possibilities with the same material composition. Furthermore, HEC is well known for its pharmaceutical use and has a low allergenic potential.

Of the other gel formers often used in the pharmaceutical industry, HEC is the only one that can be steam sterilized, as shown in the attached example. This sterilization process increases the viscosity of the gel, so that this "viscosity gain" may be considered when preparing the composition of the present invention. Through the water reabsorption of the product from interstitial body water under additional influence of body / skin temperature, the product can float up at the application site. Through this process of passing through various viscosity levels to aqueous, the phages are released and, as they continue to spread at the site of application and in ever smaller doses, act beyond:
Another reason for the use of hydroxyethyl cellulose is the low sensitivity of the gel former and consequently of the finished product to changes in pH and to electrolytes. HEC gel production and equally phage HEC gel production are easy, cheap and fast to realize both in manual manufacture and industrial scale handling. No special equipment or surfaces are required.

The hydrogel of the present invention preferably comprises 3 to 10 % HEC, more preferably 5 to 8 % HEC or 6 to 7 % HEC, most preferably 6,5 % HEC.

The hydrogel of the present invention may be prepared by mixing the individual components and adding water to obtain the final concentration. In principle, the preparation of HEC hydrogels is known in the art.

Gel manufacturing is performed manually or in technical processes and is known in the art. For example, the dissolving ingredients may be dissolved in a first part of water. Successively HEC may be incorporated in the water under moderate stirring. At least the rest of needed water may be given into the gel mass under further moderate stirring. After preparing the hydrogel, the hydrogel may be sterilized, preferably steam sterilized.

The bacteriophages may be given into the gel for example in form of a bacteriophage solution. Mechanical forces may be needed to dissolve the phages into the gel matrix. That may be done by stirring the composition while adding the bacteriophage solution or by a two-syringe-technique: Therefore one syringe may contain the bacteriophage solution, a second may provide the gel. Through a connector the gel and bacteriophage solution may be brought together, by first pressing the phages solution into the gel. The process may be replied multiple times vice versa.

The hydrogel further comprises CaCl₂ and glycerol.

Calcium serves to stabilize and thus maintain the activity of the bacteriophages. Calcium is added to the composition in the form of readily soluble calcium chloride. By dissolving the CaCl₂ in the composition, especially in the contained water, the calcium is made available to the phages as calcium ions (Ca²⁺). Calcium chloride has only low chemical reactivity on its own, does not affect the adjusted product pH and, in the quantities described below (for example in a concentration between 10 % and 33 %), has no relevant influence with regard to osmotic processes.

The hydrogel of the present invention may comprise 10 to 33 % CaCl₂, preferably 15 to 25 % or 17 to 23 %, and more preferably 20 to 22 %, most preferably 21.5 %.

Glycerol is used to increase the adhesive properties and to retard product liquefaction under the influence of body temperature and fluid, since it may serves as a lubricant, spreading agent and adhesion promoter. Glycerol is also hygroscopic and thus not only supports systematic degradation and phage release over the surface of the applied PhaTEC-drug, but it also contributes to residue-free degradation of the product. As in the case of surgical sutures, the inclusion of glycerol makes it a self-absorbable product.

The hydrogel of the present invention may comprise 6 to 22 % glycerol, preferably 8 to 18 %, more preferably 10 to 16 % or 12 to 15 %, most preferably 13.5 % glycerol.

Glycerol may be added to the composition in the form of glycerol 85 %.

In a further preferred embodiment, the hydrogel of the present invention further contains a buffer. Buffers suitable in the context of the present invention include Tris-HCl (pH = 7.4), sodium chloride and magnesium chloride.

The hydrogel of the present invention may further comprise other salts like sodium chloride, magnesium chloride, calcium chloride and potassium chloride.

The hydrogel of the present invention may preferably have a physiological pH, for example a pH of 7, 7.2, 7.4, 7.6 or 7.8. Using a buffer to ensure a physiological pH may also be helpful to attenuate the pH differences in infected, inflammatory tissues.

In an especially preferred embodiment, the hydrogel of the present invention comprises a buffer comprising sodium chloride, magnesium chloride (x 7 H2O) and Tris-HCl, pH = 7.4.

The HEC stock solution for preparing the HEC hydrogel of the present invention may comprise as an example (based on 100 g):

| | |
|---|---|
| HEC (Hydroxy-ethyl-cellulose): | 13 g = 13 % |
| CaCl₂-Solution*: | 43 g = 43 % |
| Glycerol 85 %: | 27 g = 27 % |
| Water: | 17 g = 17 % |

In a further preferred embodiment, the hydrogel of the present invention comprises
10² pfu/ml to 10⁸ pfu/ml bacteriophages,
5 to 8 % HEC,
10 to 33 % CaCl₂, and
6 to 22 % glycerol.

In a preferred embodiment of the present invention, the hydrogel of the present invention comprises
10⁴ pfu/ml to 10⁸ pfu/ml bacteriophages,
3 to 10 % HEC,
15 to 25 % CaCl₂, and
8 to 18 % glycerol.

In a more preferred embodiment of the present invention, the hydrogel of the present invention comprises
10⁸ pfu/ml to 10⁷ pfu/ml bacteriophages,
6.5 % HEC,
21.5 % CaCl₂, and
13.5 % glycerol.

The water used in the composition of the invention may be Aqua ad injectabilia.

In an exemplary embodiment, the hydrogel of the present invention is prepared using the following ingredients.

| | ***defined ingredient used*** | **percentage (by weight) [%] used** |
|---|---|---|
| **Bacteriophages** | *Bacteriophages solution containing a concentration of 10⁷ pfu* /*ml* | 50 |
| **Hydroxy-ethyl-cellulose (HEC)** | *Natrosol 250 HX* | 6,5 |
| **CaCl₂** | *CaCl₂-Soltution containing a concentration of 73,5 g* /*L* | 21,5 |
| **Glycerol** | *Glycerol 85 %* | 13,5 |
| **Water** | *Aqua ad injectabilia* | 8,5 |

In a preferred embodiment, the hydrogel of the present invention has a viscosity of 1000 mPas up to 100.000 mPas; preferably 10.000 to 80.000 mPas, more preferably 40.000 to 80.000 mPas or 40.000 to 60.000 mPas or.

In a preferred embodiment of the present invention, the hydrogel of the invention is sterile. Preferably, the hydrogel has been steam sterilized.

Sterility may be achieved by sterilizing the HEC hydrogel and the bacteriophage solution separately, or by sterilizing the hydrogel of the present invention, i.e. the hydrogel comprising hydroxyethyl cellulose (HEC) and bacteriophages.

In a further aspect of the present invention, the invention relates to the hydrogel of the present invention for use in a method of treating or preventing a bacterial infection. Disclosed is a method for treating or preventing a bacterial infection in a subject, wherein the hydrogel of the present invention is applied to the subject.

The subject may be any animal, but preferably, the subject is a mammal, more preferably a human.

The hydrogel of the present invention can be used to treat and prevent a bacterial infection. According to the invention, the term "treatment" means that bacteria, which are already present, are counteracted by the bacteriophages contained in the hydrogel, while the term "prevention" means that the bacteria are not yet present when the hydrogel is applied.

The hydrogel may be applied to any accessible surface of the subject. This includes but not limited to the skin or any accessible mucosa of the subject. The hydrogel of the present invention is especially suitable for treating a bacterial infection in a wound.

The hydrogel may be applied by any possible means. These include, but are not limited to manual application, application through a syringe, through a needle, through a catheter. Distribution is mainly done by hand or any helping device to cover the sites /areas to be covered by the product.

In addition, the hydrogel may be applied to any implant which is to be attached or inserted into the body of the subject or which has already been inserted into the subject.

According to the invention, the implant may be a joint prosthesis, vascular prosthesis, heart valve, catheter, an artificial heart, a dental implant, and a minimally invasive heart valve TAVI.

The medical areas where the hydrogel of the present invention may be applied include, but are not limited to surgery, orthopedics, radiology, nephrology, diabetics for example the superinfected diabetic foot syndrome, dermatology, cardiology, wound care, nursing facilities/services, and ambulance service.

Possible indications for the hydrogel of the invention are for example surgical sites, implants, anastomosis, wounds, for example deep wounds or wound holes, abrasions, cuts, stabs, non-intact skin tissue and mucosal tissue, decubitus / pressure ulcer, entrance points of catheters, e.g. ureter- / IV- catheter, covering of sterile material, wound and implant coverage, and suture material.

In a preferred embodiment of the invention, the hydrogel of the present invention is applied during surgery.

It has been shown in the present invention (see Examples 2 and 3) that the hydrogel is of the invention is especially useful for treating and preventing a bacterial infection when applied during surgery.

During surgery, the hydrogel of the invention can be applied by all possible means. For example, the bacteriophage gel can be applied manually and distributed in the same way, mainly by the surgeon while surgery or by any other person, especially when applied outside of the body, e.g. to wounds or skin.

Moreover application and distribution at / on the desired site /area can be done by all helping devices, mostly syringes, needles and catheters. So it is possible to apply the bacteriophages gel at minimal invasive interventions or in body / wound holes.

Because of the viscosity and the possibilities to adapt the viscosity to the circumstances found e. g. while surgery, especially when the two-syringe technique is used, and to the robustness of the product, which is provided by the duo of galenic and bacteriophages, all possibilities of application can be used.

In an especially preferred embodiment of the present invention, as already mentioned above, the hydrogel of the present invention is used to treat and / or prevent a bacterial infection of an implant. In this embodiment of the invention, the hydrogel of the invention can be directly applied to the implant, either outside the body or in the body, e.g. during surgery.

The invention is further illustrated by the attached examples and figures, which are intended to explain, but not to limit the present invention.

### Figure Legends

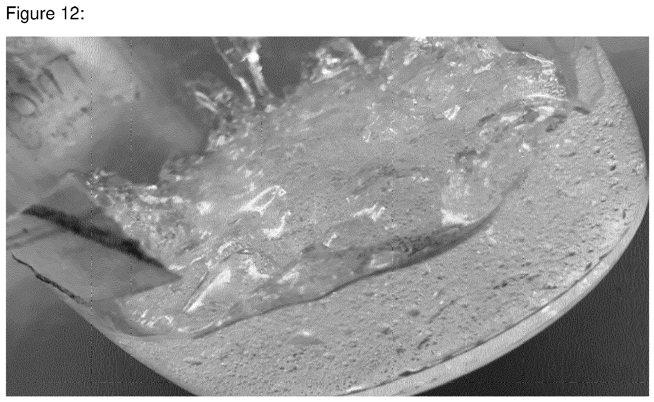

Figure 1:
   Na-Hyaluronat gel on filter before stored for 24 h at 37 °C. The gel is much too firm to be spread well on the filter paper and shows an agglomerate as a result of the stiffness of the gel.
Figure 2:
   Na-Hyaluronat gel on skin: the gel shows stiffness even on the skin. Tries of application and spreading on the shown arm were painful, due to the firm condition of the gel.
Figure 3:
   Carbomer gel on skin: the gel shows a highly adhesive character. Spreading on the skin difficult. Moreover, application and spreading was painful, due to the stickiness. A wanted layer of gel has not been applicable.
Figure 4:
   Carbomer gel: to show the stickiness of the carbomer gel a bit of gel has been taken between two fingers. The fingers then has been spread. As result the gel sticked to the fingers and got thinner in the middle. So it has been show that application was too difficult and the gel is too adhesive.
Figure 5:
   HEC gel on filter before stored for 24 h at 37 °C: the gel shows a solid character and a good spread-ability / possibility of application at the same time.
Figure 6:
   HEC gel on skin: on skin the application has been tested. The application has been very easy. Moreover a wanted layer of gel has been possible to applicate.
Figure 7:
   HEC gel after 24 h at 37 °C: the gel has been dried by the storage-conditions. The solid character after the storage, without any packaging protecting the gel from water evaporation, shows the essential of adequate primary packing. Moreover the gel stayed in the form in which was stored showing an ongoing coverage of the application area.
Figure 8:
   Polaxamer gel after 24 h at 37 °C; the gel has been dried by the storage-conditions. The solid character after the storage, without any packaging protecting the gel from water evaporation, shows the essential of adequate primary packing. The gel did not stay in the form in which was stored showing a change in the coverage of the application area and a transformation into a cube-like form.
Figure 9:
   Na-Hyaluronat gel after 24 h at 37 °C: the gel has been dried by the storage-conditions. The solid character after the storage, without any packaging protecting the gel from water evaporation, shows the essential of adequate primary packing. Moreover the gel did not stay in the form in which was stored showing a change in the coverage of the application area. Stickiness has increased with the loss of water.
Figure 10:
   HEC gel after sterile manufacturing: direct loading method, Ph. Eur. 2.6.1, showing no sign of bacterial contamination after a prolonged testing to increase the probability of detection.
Figure 11:
   Plaques could still be detected after steam sterilization of Phages: On a standardized bacterial bio burden phages has been spread out after steam sterilization. After the time of incubation plaques show that phages have survived the steam sterilizing process and are still active.
Figure 12:
   Sterile preparation of HEC gel: The Figure 12 shows the sterile result of the combination of phages and gel.
Figure 13:
   Gel after steam sterilisation: direct loading method of Ph. Eur 2.6.1, showing no sign of bacterial contamination after a prolonged testing to increase the probability of detection.
Figure 14:
   Preoperative PET-CT. Representative slides from the pre-operative [F18] fluorodeoxyglucose PET-CT scan. The enhancement as the level of accumulation of the tracer substance around the stent-graft in the thoracic aorta is a sign of active inflammation.
Figure 15:
   Intraoperative images from the first step of treatment: The descending aorta has been mechanically debrided from periaortic infectious tissue
Figure 16:
   Intraoperative images from the first step of treatment: Two endo-sponges had been placed around the aorta.
Figure 17:
   Intraoperative images from the first step of treatment: Finally, another vacuum sponge was applied for ventral coverage of the aorta (C) before the thorax was closed.
Figure 18:
   Off-the-shelf release of an aortic stentgraft system and manual surface covering with bacteriophages gel.
Figure 19:
   Lateral X-Ray after stent implantation, showing a complete overcovering of the infected stent graft by the new ones.
Figure 20:
   PET-CT three months after implanting the bacteriophage gel covered stent showing no revealing signs of infection in and around the thoracic aorta.
Figure 21 :
   PET-CT showing the infected aortic-bi-femoral vascular graft with right femoral tissue infection.
Figure 22:
   Impressions of the surgical procedures and the application of the bacteriophages gel
Figure 23:
   PET-CT three months post intervention without signs of infection enclosing the aorta or both femoral regions

### Example 1

### Finding a Suitable Phage Containing Gel- Galenic for Clinical Use: a Laboratory Trail

### Abstract

In vitro laboratory tests were carried out to find a suitable basis-gel for the PhaTEC Phages Gel-Product. The shortlist of gel formers included Hydroxy-Ethyl-Cellulose (= HEC), Sodium-Hyaluronate, Polaxamer, Carbomer and Silicon dioxide (= SiO₂ [highly dispersed]). Testing was performed according to spreadability on filter paper and on the skin, the haptics and sensory properties, as well as the gel consistency and the behavior after 24 h at 37 °C. As bacteriophages we used a cocktail of different Bacteriophages (SniPha 360) which can be purchased from the company "Phage24" in Austria. The requirements are placed on the gel, which is to become the subsequent basis of the PhaTEC Product. All tests were carried out under sterile conditions in the laboratory of Pohl-Boskamp GmbH & Co. KG, taking GLP (good laboratory practice) into account.

With regard to sterilization, in our experiments various methods are shown that lead to a sterile product of the HEC gel and the phage cocktail.

Furthermore, both the HEC gel and the phages can be steam sterilized. Thus, the PhaTEC product, containing the gel and phages, can be sterilized in the final container for easy and cost-effective industrial production.

### 1. Background

### 1 . Stability and Spreadability

The PhaTEC product is to be used for intra- and extra-corporal application. Among other things, the product is to be applied to mucous and skin areas, wounds and surgical sites such as anastomoses. For this purpose, an appropriate spreadability is required (to cover an application area) with simultaneous product stability - for example, to generate / apply an individual layer thickness and to remain at the place of application. Moreover, the gel should be spreadable so that it can be applied easily and painlessly to sensitive areas such as fresh wounds. In addition, cooling is desirable, especially in the case of extra-corporeal application, in order to counteract inflammatory reactions and to provide a pleasant feeling on the application area.

### 2. Possibility of Sterilization

Due to the pursuit of a product that is intended for extra- as well as intra-corporeal application, the product and thus the initial components, such as the base gel, have to provide the possibility of sterility.

### 3. Adequate spreading around the place of application

The gel should be reduced in viscosity by the introduction of skin or body temperature and body water, so that the phages contained later are released over a defined time. This results in a prolonged release of the therapeutic agent, which does not affect the body, due to the degradation of the product by dilution and physiological excretion processes.

### 4. Feeling on the skin

To all medical, as well as pharmaceutical requirements, the acceptance of the patient is essential. Since the phages do not cause any physical reactions and have no side effects, the gel, which the patient feels, especially when applied cutaneous / extra-corporal, must be pleasant on the skin and / or mucous membrane.

### 2. Gel-Manufacturing

Initially, all gels were prepared with a gel former content of 13 %. The Carbomer, Na-hyaluronate and HEC gels were the easiest to prepare.

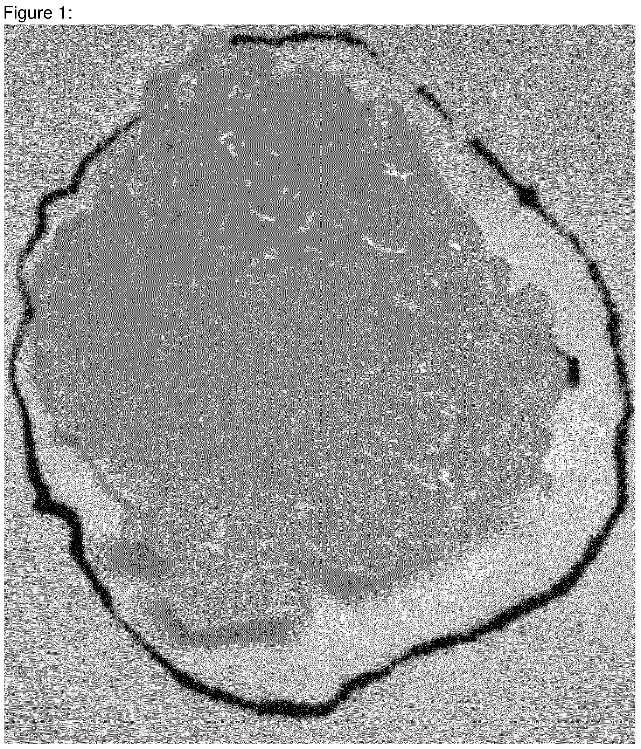

From the five preparations, only three solid gels were obtained:
HEC-, Carbomer- and Sodiumhyaluronate-Gel. The Silicon dioxide -at the applied concentration- formed a viscous liquid but not a solid gel and did not dissolve completely. The Polaxamer showed gel-islands that were not connected with each other; in addition, much water was not absorbed (Fig.1)

### 3. Haptic / Sensory Skin Testing

### Methods:

Different Gel-formations had been tested at the skin surface of the same test person. Sensitive feeling as far as skin reaction had been observed.

The Na-hyaluronate (Natriumhyaluronat EP) gel was much too firm to be spread well on the filter paper. Figure 1

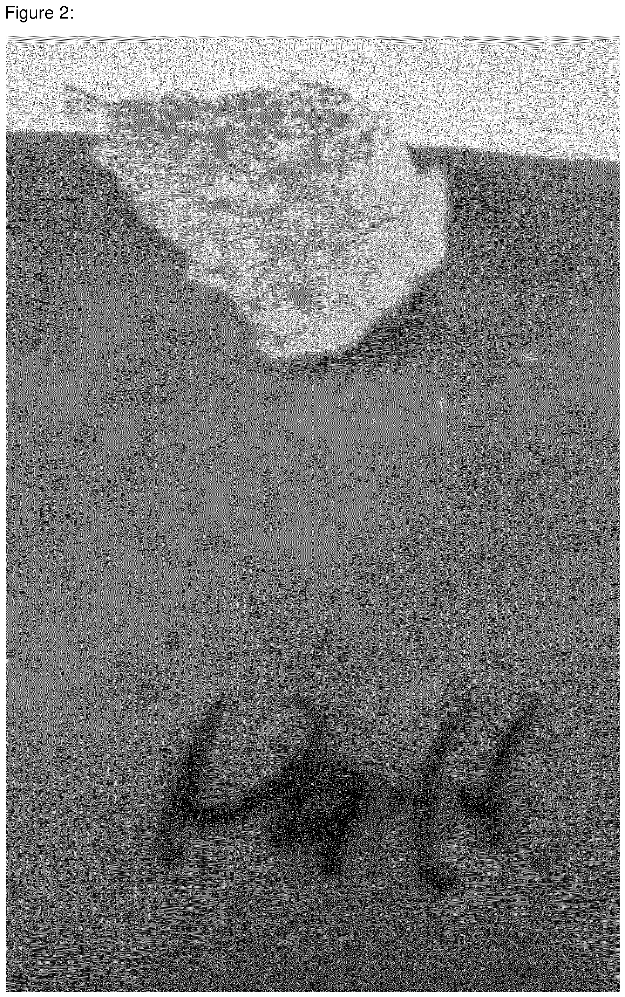

\On the skin, especially on hairy skin areas such as the arm used in the test, application and spreading was painful, due to the firm condition. Figure 2

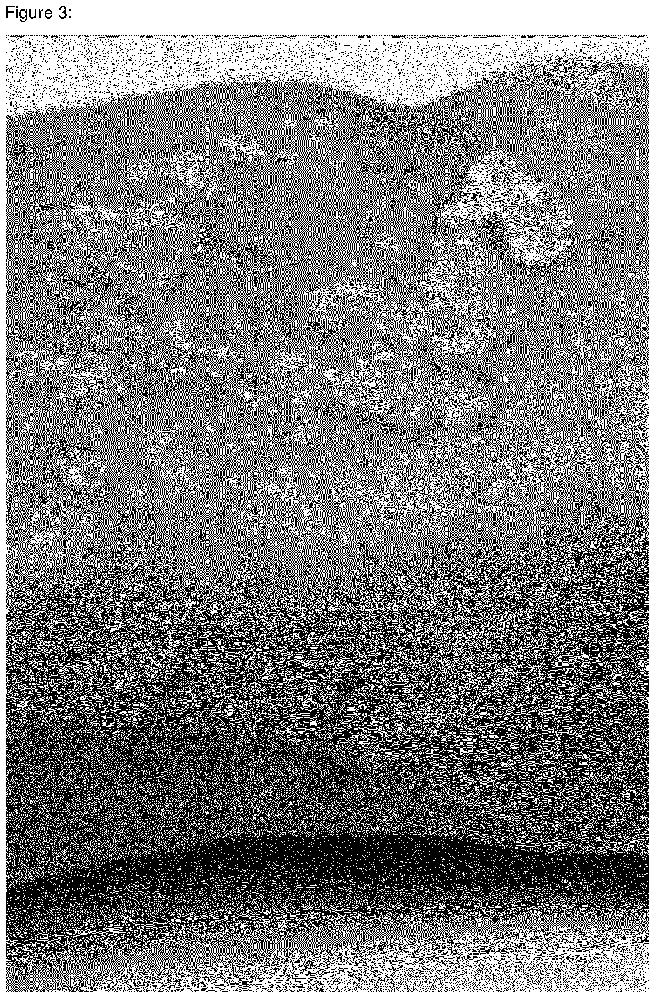

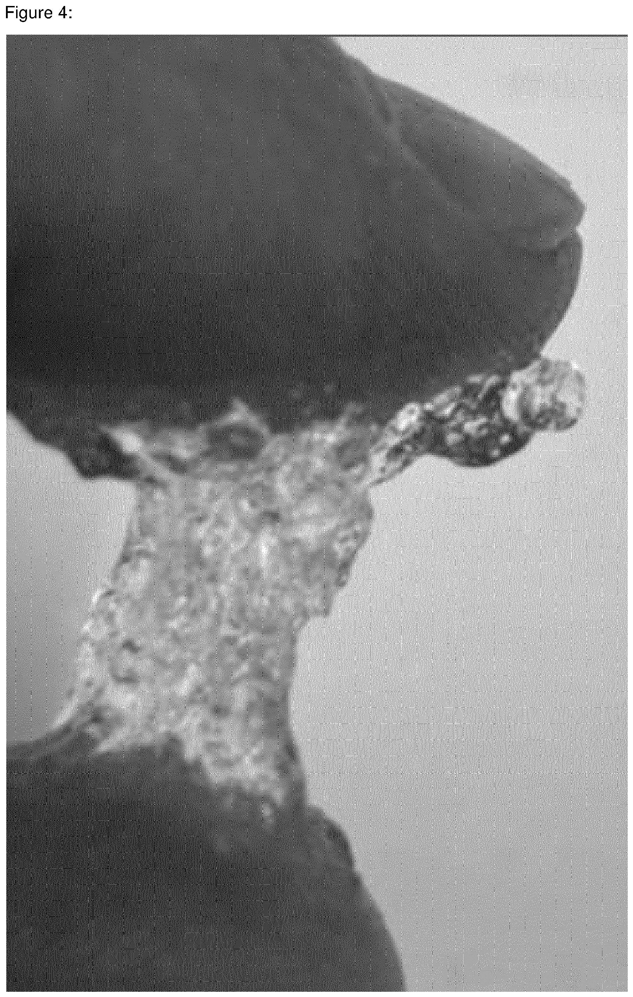

The Carbomer (Carbomer EP; Carboprol 71 G) gel was highly adhesive and therefore difficult to spread on the filter paper and almost impossible to spread on the skin. Moreover, on the skin, especially on hairy skin areas such as the arm used in the test, application and spreading was painful, due to the stickiness: Figure 3, Figure 4

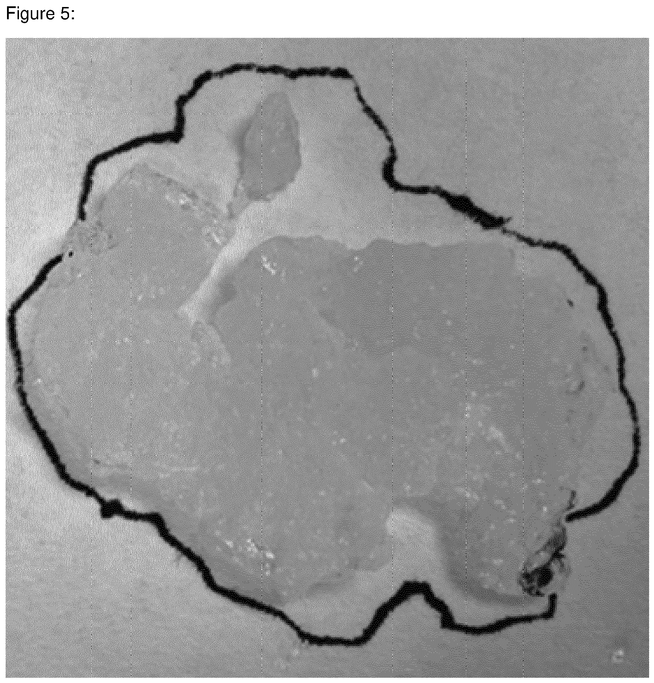

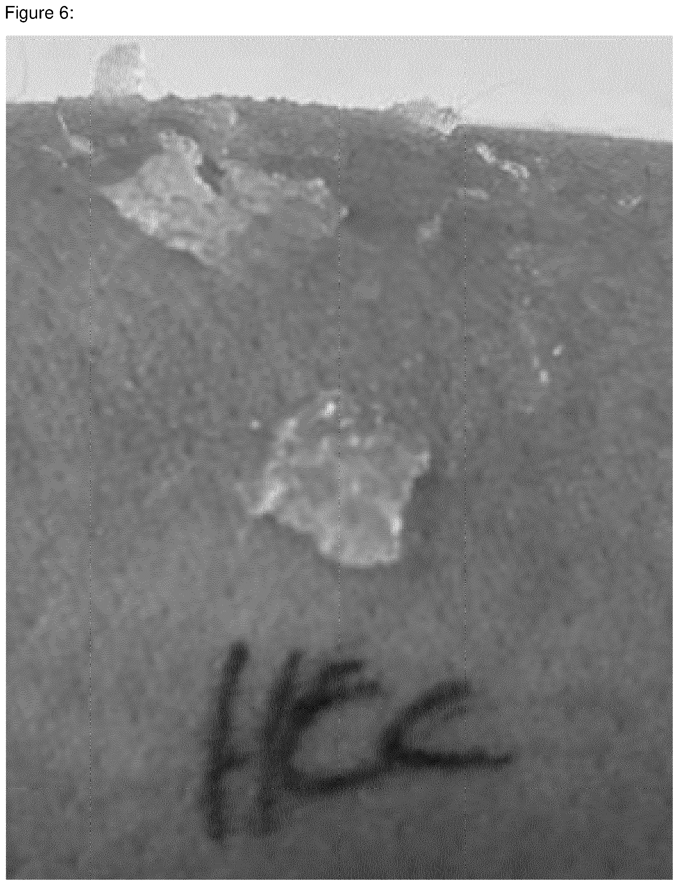

The HEC (Natrosol 250 HX) gel felt the most pleasant: It was easily spreadable on the filter paper and on the skin with a simultaneous firmness that allowed application of individual layer thicknesses: Figure 5, Figure 6

### Results:

The feeling of the gels proved to be very different: The gels showed very different characteristics: If the SiO₂ (hochdisperses Siliciumdioxid; Aeroperl 300 Pharma) and Polaxamer (Polaxamer 407) gel were not tested on the filter paper and / or skin due to the liquid state of the SiO₂ gel and due to the island formation of the Polaxamer gel, the Carbomer gel was found to be highly adhesive and difficult to apply because of the free flowing properties. The stickiness of the Carbomer gel was very unpleasant when applied to the skin and painful in hairy areas, such as (in the test) the arm. The cooling effect was almost immediately noticeable. The gel, especially on the skin, did not remain at the application site and immediately flowed undirected and uncontrolled into the surrounding area. An appropriate layer thickness could not be applied.

The Sodiumhyaluronate gel was much too firm for painless, simple application: It could hardly be spread on the filter paper and on the skin, especially in hairy areas, such as (in the test) the arm, it was painful and difficult to spread due to its firmness.

Hard, but still easy and painless to apply and remaining at the application site as desired, was the HEC gel: Here, it was not only possible to demonstrate good spreadability on the filter paper and on the skin; the feeling on the skin was also pleasant. Application on the skin was simple and painless. The gel distribution could be done individually with regard to the desired layer thickness. The cooling effect occurred after some time due to the applied layer thickness. In addition, the applied gel layer gave a feeling of a protective layer, like a plaster or similar.

### 4. Gel-galenic behavior under drying conditions

The heat exposure of 37 °C was carried out by means of a drying oven. In order to test the cooling effect desired when external applied, the gels (10 ml each) were placed in the center of a filter paper and the border was marked. The gels were stored at 37 °C for 24 h in order to demonstrate the drying of the gels and thus a cooling effect due to evaporative cooling.

### Methods:

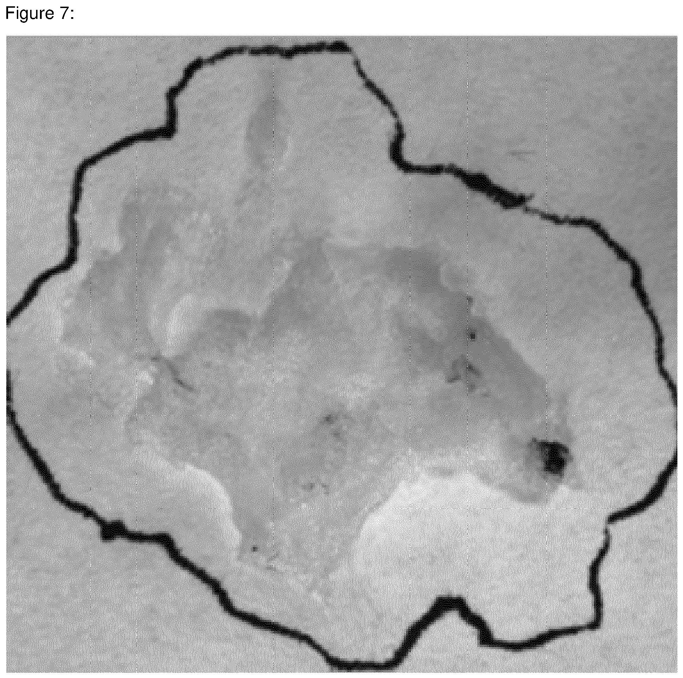

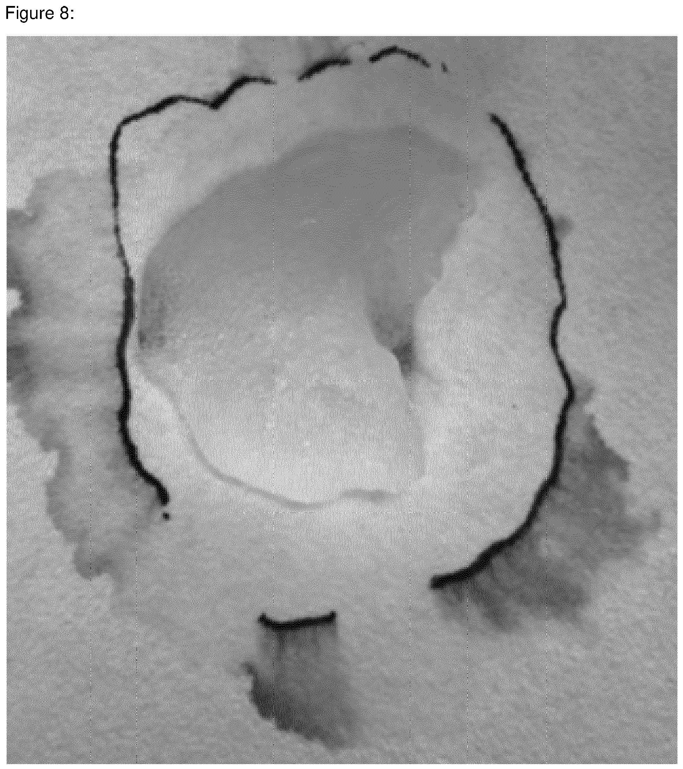

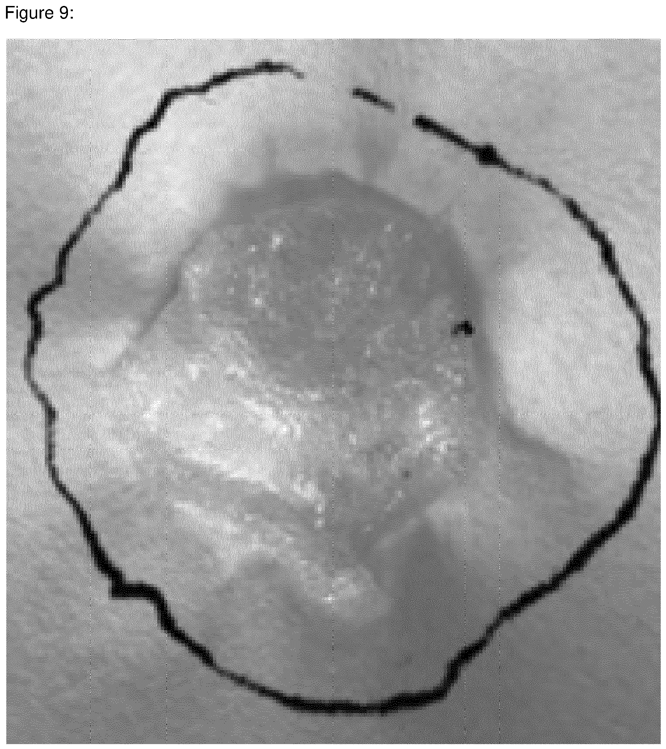

The haptic/sensory test after 24 h at 37 °C was performed with gels that were in cups with the lid on in the drying cabinet. Figure 7, Figure 8, Figure 9

### Results:

The primary packaging provided adequate protection against dehydration. Under physiological conditions, the product is liquefied by the interstitial water and physiologically degraded.

### 5. Sterility

### 5.1. Sterile production

### 5.1.1. Gel

### Methods:

The gel was sterilely prepared in laboratory-scale tests. For this purpose, previously sterilized equipment and a Bench (laminar air flow) were used. The prepared HEC gel was tested according to the direct loading method of Ph. Eur 2.6.1.(Fig.5).

### Results:

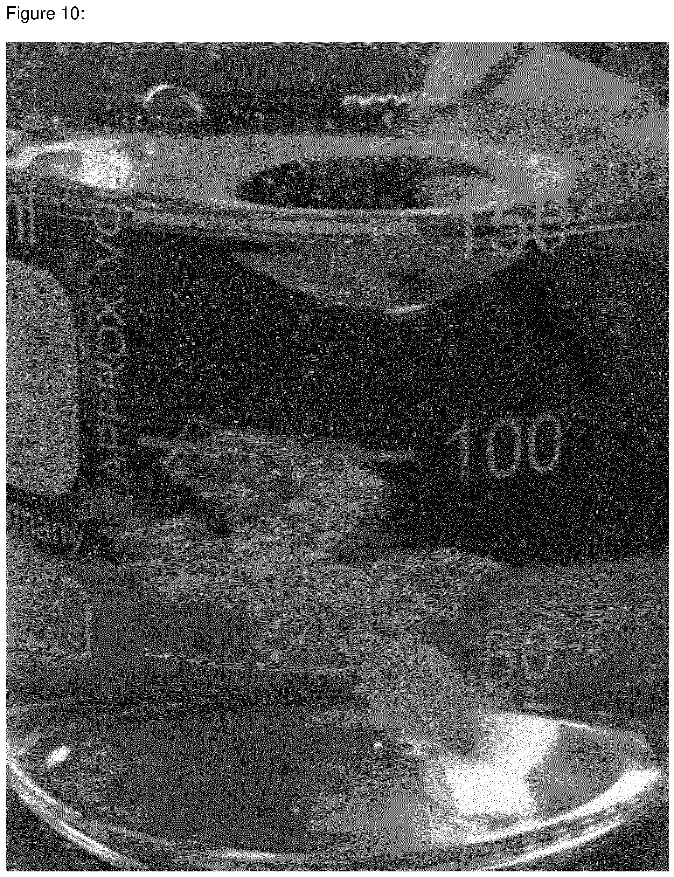

No bacterial growth after 24 h until 9 days, when the extended test was ended. No microbial growth was evident over the entire period. Figure 10

### 5.1.2. Phages

It is a defined objective of PhaTEC that its phage products can be produced industrially, standardized and easily. A major aspect of a simple, industrial production is the sterilization of the product in the final container. Two possibilities of sterilization of the gel are described above and have been verified by the corresponding tests. The phages can be sterilized via sterile filtration, as tested and described earlier.

### Methods:

Phages were sterile-filtered and showed no bacterial growth on different culture media after 24 , 48 and 72 h at 37 °C. The cocktail was tested positive on an E. coli bacteria culture on agar, Samples of the phage cocktail, containing about 10⁷ pfu /ml, were steam sterilized and carried out the test with the steam sterilized BPG against the same bacterial strain on fresh corresponding agar.

### Results:

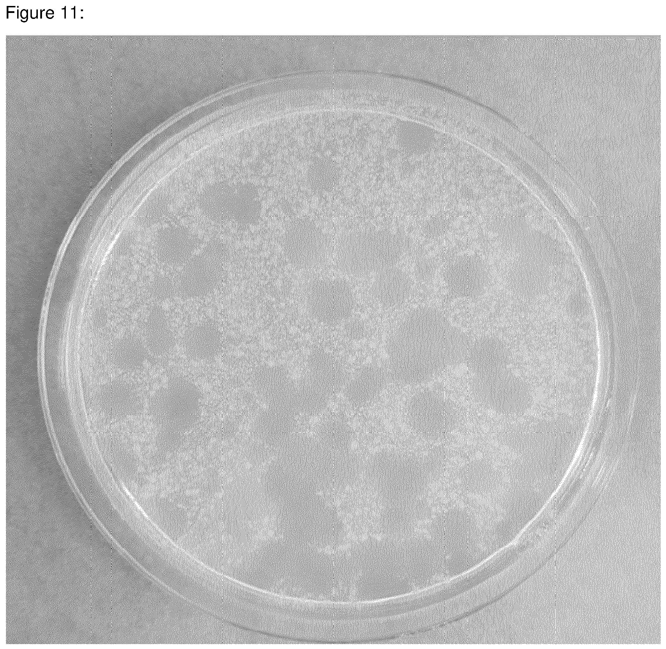

Plaques could be detected, approx. 6 x 10^2 PFU/ml. That leads to the result, that steam-sterilized bacteriophages from our phage cocktail still have lytic activity against the corresponding bacterial strain. Figure 11

In conclusion it has been demonstrated, that Steam-sterilization of phages ispossible.

### 5.2. Steam sterilization

### 5.2.1. Gel

### Methods:

The gel was prepared, in addition to sterile manufacture, in a normal laboratory clean setting and steam sterilized in the final container. Figure 12

### Results:

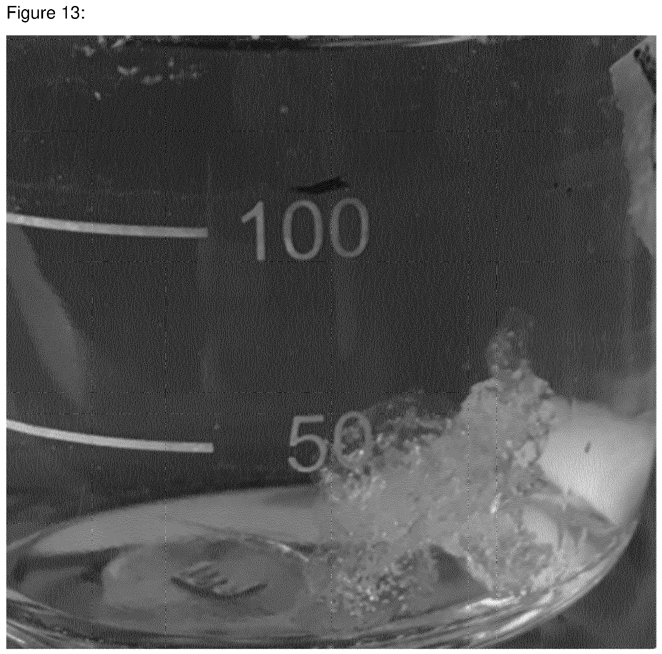

The steam sterilized HEC gel showed no growth over the entire time when tested as described under "Sterile Preparation". Figure 13

### 6. Summary

Among the most suitable gel formers tested, Hydroxy-Ethyl-Cellulose is the best gel former: the haptic, the good, uniform, even protective feel on the skin, as well as the properties of easy application, also in terms of a desired layer thickness, are just some of the aspects that lead to this result.

With regard to sterilization, there are various methods shown that lead to a sterile product of the HEC gel and the phage cocktail. All individual tests lead to the conclusion that all methods and procedures, presented above, are suitable to bring the PhaTEC Product into a sterile state.

Furthermore, both the HEC gel and the phages can be steam sterilized. Thus, the PhaTEC product, containing the gel and phages, can be sterilized in the final container for easy and cost-effective industrial production.

### Example 2

Successful treatment of an infected TEVAR with extra- and endovascular bacteriophage application

### Abstract

Objectives: Graft infections are severe complications in vascular surgery. Surgical resection of infected aortic stent-grafts is associated with high mortality and morbidity. Therefore, alternatives to inadequate antibiotic treatment and extensive surgery are urgently needed.

### Case

A 67-years-old woman was admitted with an infected stent-graft in the thoracic aorta. Local infection was confirmed by PET-CT imaging. Due to comorbidities, surgical resection of the stent-graft was not feasible. Therefore, a three-step approach for local bacteriophage treatment was performed as a last-resort treatment. First, the para-aortic tissue was debrided via left thoracotomy, a bacteriophage suspension was applied around the aorta and an irrigation-vacuum-system was installed. After repeated alternating instillation of the bacteriophage suspension for three days, as the second step vacuum sponges were removed and a bacteriophage-containing gel was locally applied locally around the aorta. In the third step, the bacteriophage-containing gel was applied to a thoracic stent-graft, which in turn was endovascularly placed into the infected stent. After 28 days, the patient was discharged from hospital with normalized infection parameters. PET-CT imaging at three months post intervention did not show signs of infection in or around the thoracic aorta.

### Conclusions:

This case demonstrates successful treatment of an infected endovascular stent-graft by application of bacteriophages both to extravascular and, as a novel approach, endovascular sites using a bacteriophage-coated stent-graft.

### Keywords:

staphylococcus aureus sepsis, graft infection, phage therapy, antibiotic resistance

### Background

In vascular surgery, infections of vascular grafts are severe complications. Especially infections of endovascular aortic stent-grafts are associated with a high morbidity and mortality up to 75 %. Since these endovascular procedures are often performed in older patients with multiple comorbidities who do not qualify for open aortic repair, the required removal of the infected stent-grafts and *in situ* reconstruction with autologous tissue or extra-anatomic replacement are related with early postoperative morbidity and mortality over 20 %. Even with successful treatment, the reinfection rate is up to 20 %.

Bacteria embedded into the peri-prosthetic tissue form a surface-adherent biofilm and therefore have a up to 1000-fold greater tolerance to antibiotics. *Vice versa,* even targeted antibiotic treatment can only suppress a stent-graft infection and is no curative treatment option.

In order to reduce the morbidity and mortality of the unavoidable surgical treatment, alternative less invasive approaches are urgently needed. In this context, bacteriophages and their bacteriolytic activities are a promising therapeutic option.

### Case

In August 2020, a 67-year-old female patient was admitted to the hospital due to worsening general condition and thoracic respiratory pain. There was a pronounced cough with deep inspiration, without sputum, and fever up to 38.6°C. Infection with Sars-CoV-2 was ruled out. The patient showed a leukocyte count of 16.7 x 10⁹/l and a serum C-reactive protein value of 199.6 mg/l. Secondary findings included a condition after thoracoabdominal stent graft implantation (COOK stent 34/152 mm) after Stanford type-B aortic dissection in February 2009. Furthermore, the patient suffered from Osler disease that required prednisolone treatment, condition after lung artery embolism, arterial hypertension, an atrophic left kidney and diverticulosis of the sigmoid colon.

Chest X-ray showed no evidence of pneumonia. Antibiotic therapy with ampicillin/sulbactam and roxithromycin was started. After *Staphylococcus aureus* was detected in the blood culture, antibiotic therapy was switched to Flucloxacillin and after five days, to cefuroxime due to an allergic skin eczema.

Endocarditis was ruled out. The patient showed progression of known leukocytoclastic vasculitis with involvement of both legs, arms, cleavage, hands, and soles without involving the kidneys. Cutaneous symptoms improved with intensed prednisolone therapy. The progression of vasculitis was considered a reaction to the systemic infection. The antibiotic treatment was switched to meropenem and cefazolin.

A computed tomography scan of the chest and abdomen did not reveal infection foci. To rule out the aortic stent graft as a focus of infection, a fluorodeoxyglucose PET-CT was performed.

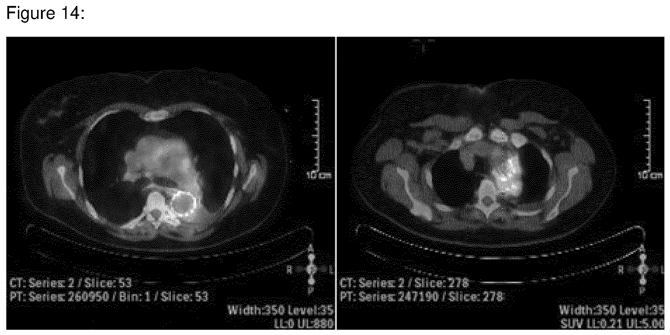

As a result, pathologically increased metabolic activity of the entire proximal aortic stent was visualized, beginning at the level of the mid-aortic arch and extending to the level of the eighth thoracic vertebra as a sign of florid stent infection. Additionally, inflammatory mediastinal soft tissue swelling and left pleural effusion were described. Figure 14.

Due to the bad condition and several comorbidities of the patient, the surgical resection of the infected stent-graft and autologous anatomical reconstruction were not feasible. The patient herself whished an alternative to indefinite systemical antibacterial treatment. Therefore, an experimental approach using local bacteriophage application was planned as a last resort treatment according to Article 37 of the Declaration of Helsinki in accordance with the local ethics committee (A 2021-0132).

### Bacteriophage treatment

As a curative therapeutic strategy a three-step approach for both extra- and endovascular application of SniPha 360 (Phage24.com, Austria) was performed. SniPha 360 is a commercially available cocktail of lytic bacteriophages against *Escherichia coli, Staphylococcus aureus, Pseudomonas aeruginosa, Streptococcus pyogenes, Proteus vulgaris* and *Proteus mirabilis.*

After the risks and benefits of the experimental procedeure were explained, the patient consented to the therapy. First, the extravascular treatment was performed by left-sided thoracotomy. The visceral pleura was found to adhere to the aorta. After obtaining local swab specimens for microbilogical analysis, debridement and jet-lavage were performed. Then, 20 ml SniPha 360, diluted in 100 ml 0.9% NaCl were dropwise applied onto the infected para-aortic tissue. Then, two endo-sponges (Endo-SPONGE^{®}, B.Braun, Melsungen, Germay) were placed on the small and large curvature of the aortic arch and the proximal descending aorta respectively, followed by a V.A.C.^{®} GRANFOAM^{™} Dressing sponge (18 x 12.5 x 3.2 cm, KCI Medizinprodukte GmbH, Wiesbaden, Germany) (Fig. 2C). The endo-sponges were connected to the V.A.C. VERAFLO^{™} Therapy system (KCI Medizinprodukte GmbH). To avoid contact of the sponges to the lunge a Suprasorb^{®} CNP Drainage film (25 x 20 cm, Lohman-Rauscher GmbH & Co., Neuwied, Germany) was placed above the sponges. The thorax was closed after placing a chest tube and the patient was refered to intermediate care unit.

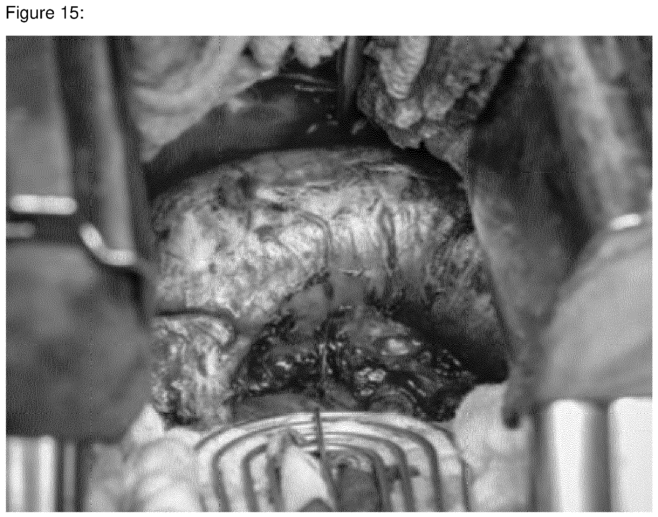

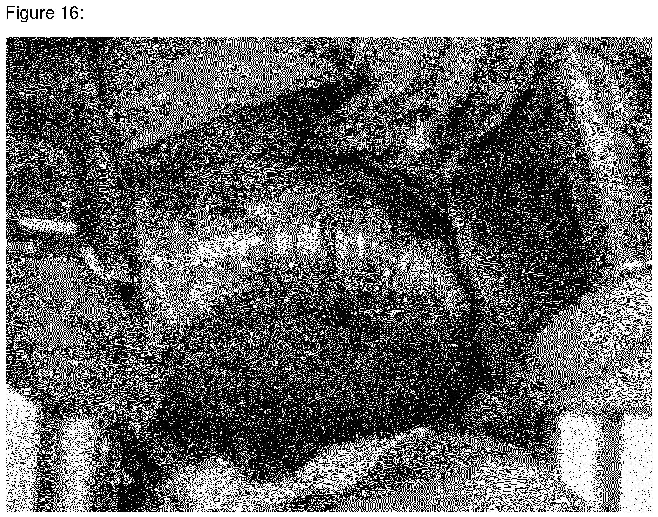

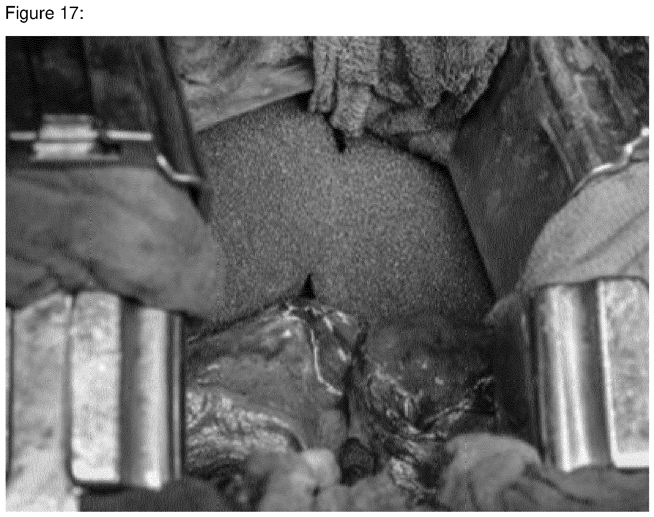

There, once daily, the intrathoracic fluid was pumped out via the V.A.C. VERAFLO^{™} therapy system two hours prior bacteriophage treatment, followed by three times flushing and draining of the sponges with 500 ml 0.9% NaCl, respectively. Afterwards, the endo-sponges were flushed with 20 ml SniPha 360, diluted in 100 ml 0.9% NaCl. Both the endo-sponges drainage and the chest tube were clamped until the next day. Figure15, Figure 16, Figure 17

As the second step, the patient was re-thoracotomized after three days. After extraction of all vacuum sponges, the aorta and its surrounding tissue were covered with 40 ml SniPha 360 incorporated in 15.8 % hydroxyethylcellulose gel. The thorax was closed and the patient was transfered to intermediate care unit.

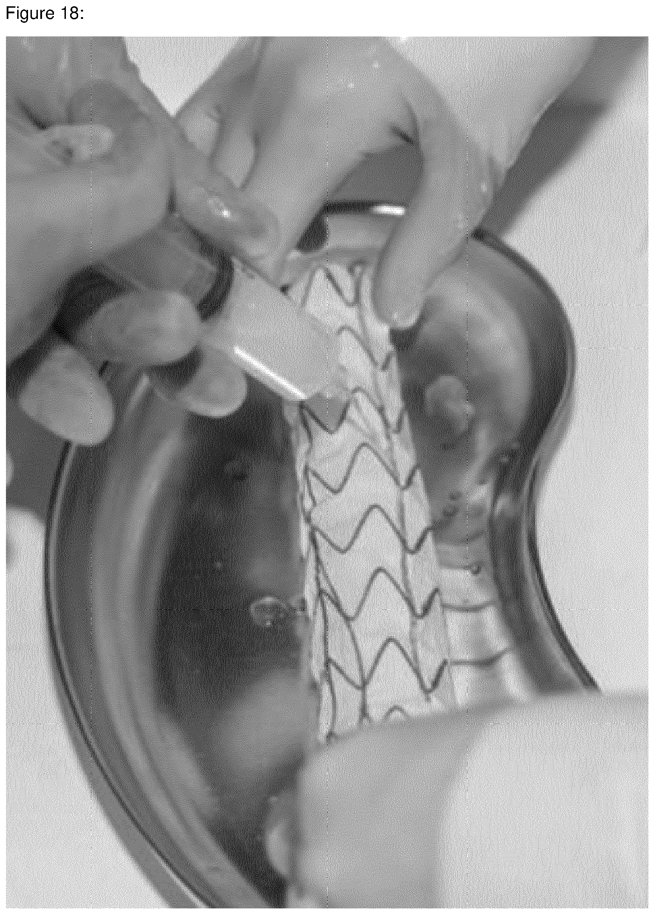

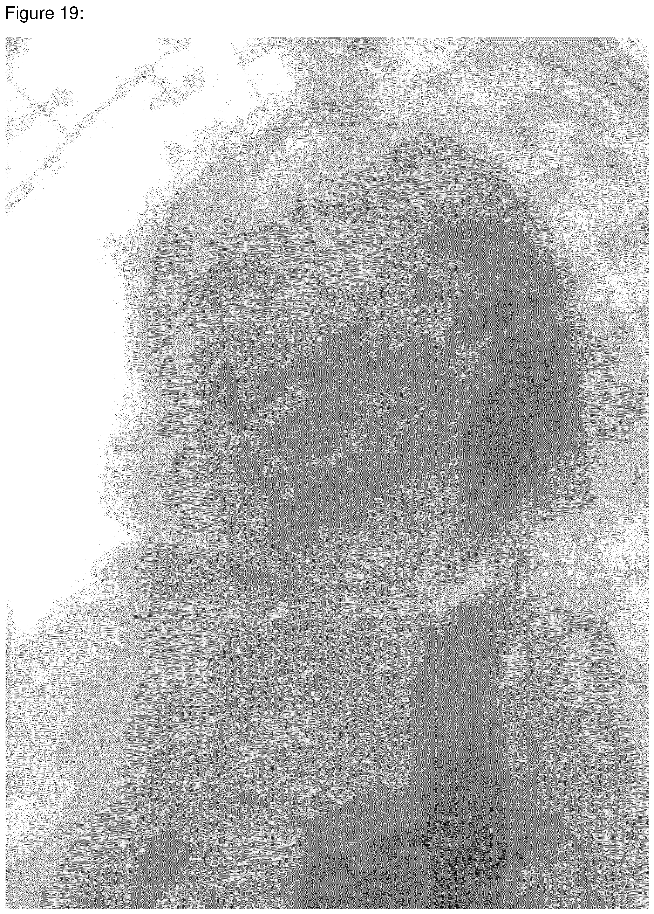

The third step was performed after another three days. Therefore, two sterile RELAY NBS PLUS stent- grafts (Vascutek Terumo-Bolton Medical, Vascutek Germay GmbH, Hamburg, Germany) were released coated with a mixture of 40 ml SniPha 360 and 15.8 % hydroxyethylcellulose gel. Afterwards the externally bacteriophage-coated grafts were re-assembled for endovascular placement. This was performed via the left common femoral artery. After overview angiography the two grafts were placed with small overlapping of the infected stent-graft. Figure 18, Figure 19

All invasive procedures were uneventful and no side effects were observed. After the endovascular treatment the patient was referred to a regular ward and recovered soon. Infection parameters decreased, the antibiotical medication was discontinued and the patient recovered. The signs of vasculitis disappeared as well. After four weeks of prolonged physio-therapeutical mobilization in hospital, the patient was discharged to follow-up rehabilitation therapy in good general condition.

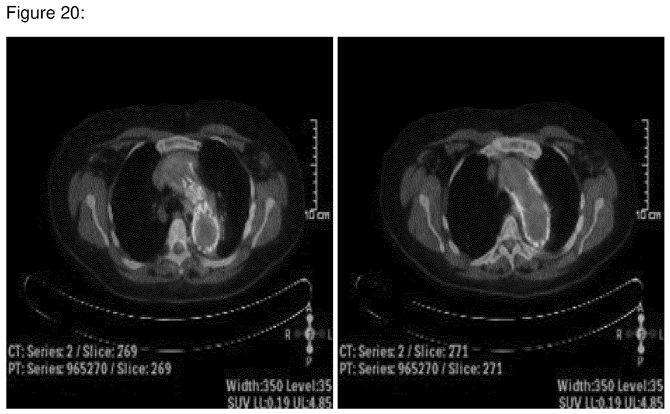

A PET-CT scan performed three months after the bacteriophage treatment did not reveal signs of infection in or around the thoracic aorta were detected. The patient recovered further while infection parameters were undetectable. Figure 20

### Discussion

This case demonstrates a successful treatment of an infected endovascular stent graft by means of local bacteriophage treatment. To our knowledge, this is the first time a stent-graft was impregnated with bacteriophages for local endovascular application.

Bacteriophages are known as a potent anti-bacterial treatment due to their lytic activity. Compared to other antibacterial therapeutic strategies like local Rifampicine treatment, bacteriophages have no cytotoxic effects on vascular cells'. In turn, they have the advantage that they act both on multi-drug resistant bacteria as well as biofilm-organized bacteria. Recently, a case series of eight patients with infections of vascular grafts, surgical wounds or implanted medical devices further demonstrated the feasibility of using different bacteriophages with lytic activity for successful treatment of bacterial infections². Although bacteriophages were used for successful treatment of infections of vascular implants, bacteriophage treatment is still not common and no officially recommended option for infections in the Westernized hemisphere.

In present case, the physical condition and the significant comorbidieties disqualified the patient for a surgical resection of the infected stent-graft and anataomical reconstrunction of the thoracic aorta. By applying bacteriophages both into the vascular lumen as well as onto the peri-prothetic tissues the graft infection with *Staphylococcus aureus* was successfully treated. The endovascular application of bacteriophages using a releasable stent-graft coated with bacteriophages and re-assembled under sterile conditions before it is insertion into the patient was an important issue of the approach. The intravascular application of phages intravascular directly to the infection site, thus assured a maximum concentration, contact time and invasion of the bacteriophages into the infected tissue.

Although multiple operative steps, including two times thoracotomy and the endovascular stent graft application, were performed, the respective distress caused by each procedure was markedly less compared to classical surgical treatment. The prolonged postoperative period at the hospital was due to the already initially weak condition of the patient, which required an extensive physiotherapeutical mobilization.

Since the physical condition of the patient significantly improved over the time and a three month follow-up PET-CT scan revealed no signs of infections, it could be assumed that the bacteriophage treatment was successful. However, there is an ongoing follow-up for the patient to assure a lasting treatment success.

In summary, this case report demonstrates that bacteriophage treatment could be a curative treatment option for patients that are not suitable for extensive surgical approaches.

### Example 3

### Successful Treatment of a Chronically Infected and Occluded Aorto-Bifemoral Dacron^{®} Bypass with Bacteriophages

### Objectives:

Graft infections are severe and dreaded complications in vascular surgery. The surgical resection of infected aortic grafts is associated with high mortality and morbidity. Therefore, alternatives to inadequate antibiotic treatment and extensive surgery are indispensable.

### Case:

A 66 year-old patient was admitted with an infection of a chronically occluded aorto-bifemoral Dacron^{®} prosthetic bypass. After various transfemoral surgical recanalization attempts in the anamnesis, there were hostile tissue conditions on both femoral sides with chronic wound infection and exposed grafts. Local infection was confirmed by PET-CT imaging. Due to the patient's comorbidities, nothing but the sole explanation of the prosthetic bypass was medically indicated and reasonable. Further we intended to treat the bilateral femoral wound healing disorder. Bacteriophages were contemplated to be an alternative therapy option for intra- and postoperative therapy of the graft related soft tissue infection. After relaparotomy, the infected aortic prosthesis was extirpated, and the aorta was sutured. Bacteriophage suspension was instilled on a Tabotamb-Snow^{®}, which was then placed retroperitoneally. After the femoral anastomoses had been discontinued, a bacteriophage-soaked fleece was placed bilaterally on the femoral side, using the same principle. The wounds were mobilized and closed without further drainage. After 10 days of inpatient stay, the patient could be discharged with subjective well-being, irritation-free wound conditions and without systemic inflammation parameters. PET-CT imaging at three months post intervention did not show signs of infection around the aorta or both femoral regions.

### Summary:

This case demonstrates the supportive antibacterial effect of bacteriophages in septic aortic surgery and the successful secondary closure of chronically infected femoral wounds in high-risk patients.

### Background

In vascular surgery, infections of the vascular grafts are considered to be severe complications. Especially infections of aortic grafts are associated with a high morbidity and mortality of up to 75 %. Since these procedures are often performed in patients with multiple comorbidities, the required explantation of the infected graft and the extensive struggle with the related abdominal infection is related with an early postoperative morbidity and mortality of even over 20 %. Despite the initial achievement of a successful treatment, the general rate of reinfection can be up to 20 % of cases. This is mainly due to bacterial colonies embedded in the peri-prosthetic tissue, which then form a surface-adherent biofilm and hence have an up to 1000-fold greater resistance to antibiotic administration. Even a targeted antibiosis appropriate to antimicrobial susceptibility testing can only suppress a graft infection but does not constitute a curative treatment option. The most common pathogenic bacteria associated with graft inflammation are Staphylococcus aureus, Staphylococcus epidermidis and other coagulase-negative staphylococci, Enterobacterales, Pseudomonas aeruginosa and corynebacteria [3]. These bacteria regularly enhance their specific virulence by attaching to the prosthetic material, and hence averting the local immune response by forming biofilms, that hinder phagocytosis. Furthermore, systemic antibiotic therapy is often inadequate due to the lack of effective saturation concentrations within the inflammatory periprosthetic tissue. In order to reduce the morbidity and mortality associated with the often inevitable surgical treatment, less invasive approaches to adequately treat the infection of the surrounding tissue are urgently needed. In this context, bacteriophages and their bacteriolytic activity are a promising therapeutic option.

### Case

In November 2020, a 66 year-old male patient was referred to the emergency ward by his general practitioner with the clinical symptom of an acute abdomen. The examination revealed ubiquitous tenderness on all quadrants with peritonism in the lower abdomen. An infection with SARS-CoV-2 was ruled out. Further examination showed an elevated body temperature of 39.2 °C, and blood testing revealed a leukocyte count of 9.4 x 109/l, as well as an elevated serum C-reactive protein of 90.2 mg/l. The chest X-ray depicted no evidence of pneumonia. An endocarditis was ruled out. Calculated antibiotic therapy with ampicillin/sulbactam was started in the usual dosage intravenously. Blood cultures were positive for Methicillin-susceptible Staphylococcus aureus. Secondary findings included the status of ubiquitous arterial occlusive disease. Due to the necessity of numerous vascular operations on both legs, the patient had eventually undergone a thigh amputation on the right side 12 months earlier; the left side revealed a chronically occluded polytetrafluorethylen (PTFE) Stockmann bypass still in situ.

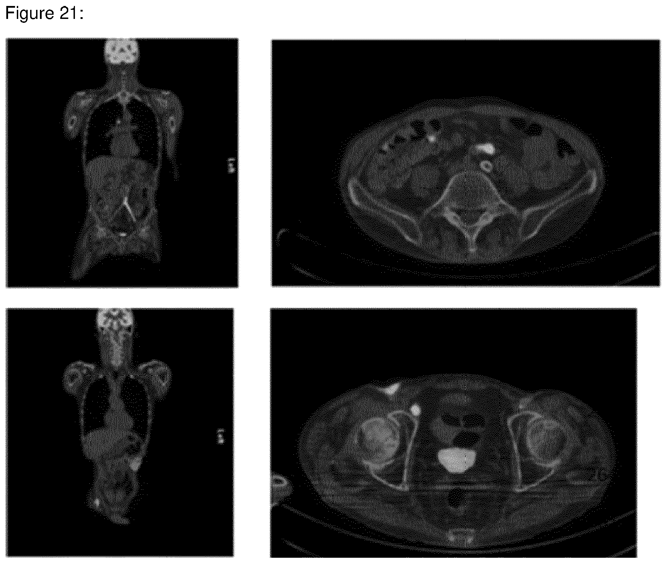

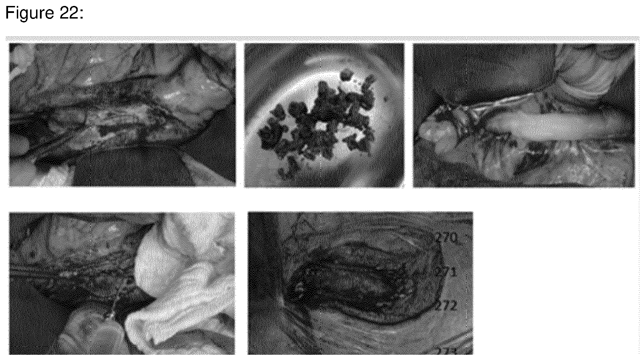

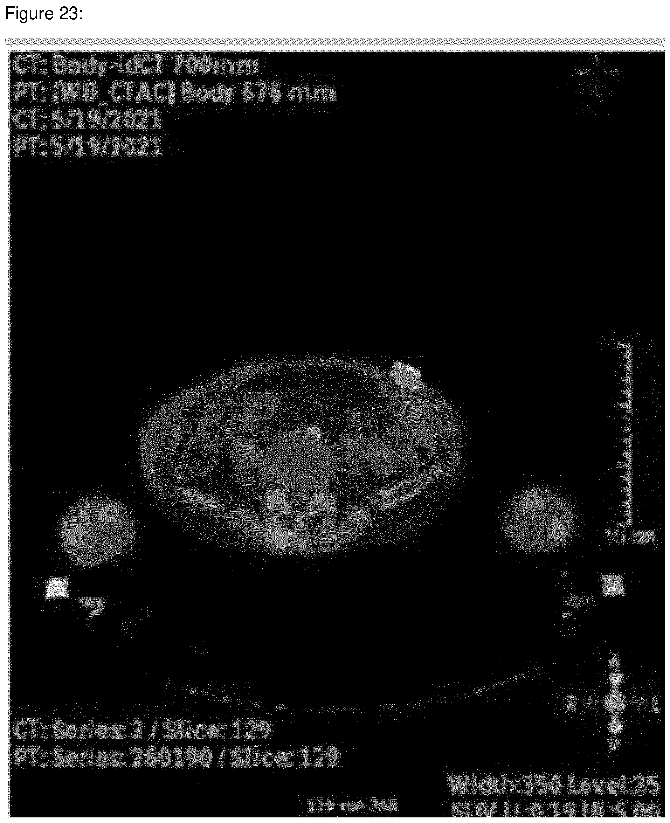

After various transfemoral surgical recanalization attempts in the anamnesis, there were hostile tissue conditions bifemoral with a chronic wound infection leading to exposed graft material. Wound swabs exposed the presence of Staphylococcus aureus and Escherichia coli, indicating a polymicrobial infection. The peripheral blood flow of the lower limbs was compensated. Initially, a CT scan of the abdomen was performed, whereupon an occluded and infected aorto-bifemoral graft was assumed. The subsequently performed PET-CT scan displayed a visibly increased metabolic activity in the area of the graft, so that we diagnosed a chronically occluded and infected aorto-bifemoral prosthetic bypass with subsequential bifemoral infections, leading to the cutaneous wound healing disorder. Figure 21

Due to the patient's comorbidities, we generally intended an operation and anesthesia time as short as possible with an efficacious treatment by explanting the prosthetic bypass. Further we planned to forego a lavage program for the septic abdomen and intended a primary closure of the abdomen. In order to treat the local inflammation in the abdominal and femoral areas in the long term intra- and postoperatively, the use of bacteriophages was considered to be plausible alternative therapy option in this case. The patient himself favored an alternative solution compared to an indefinite lasting systemical antibacterial treatment. Therefore, an experimental approach using local bacteriophage application was intended as a last resort treatment in line with Article 37 of the Declaration of Helsinki and in unity with the local ethics committee (A 2021-0208).

### Bacteriophage Treatment

As a curative therapeutic strategy an intra and extra abdominal application of SniPha 360 (Phage24.com, Austria) was executed. SniPha 360 is a commercially available bacteriophage cocktail of lytic bacteriophages against Escherichia coli, Staphylococcus aureus, Pseudomonas aeruginosa, Streptococcus pyogenes, Proteus vulgaris and Proteus mirabilis. After outlining the potential risks but also benefits of the experimental procedure, the patient consented to the therapy. When performing the relaparotomy, cloudy fluid appeared within the abdomen. After an initial lavage, the retroperitoneum was opened, and the proximal aorta was prepared for clamping. The aorto- bifemoral Dacron^{®} prosthesis presented a shell of a biofilm and was embedded in putrid fluid. The infected aortic prosthesis was extirpated, and the aorta was then sutured over. The prosthesis was retrieved femorally after mobilization of the legs of the prosthesis. The bacteriophage suspension was instilled on Tabotamb-Snow^{®}, which was placed retroperitoneally around the infection. The retroperitoneum and abdomen were primarily closed without further drainage. After removing the femoral anastomoses, the wound conditions were debrided, mobilized and lavaged with a sharp spoon. A bacteriophage-soaked fleece was then placed bilaterally on the femoral side by the same principle, and the wounds were closed again without further drainage.

The operation time was 52 minutes, without significant blood loss. Subsequently the patient could be taken to the intensive care unit and extubated without the need for catecholamines. After 10 days of hospitalization, the patient could be discharged with subjective well-being, irritation-free wound conditions and normal findings for inflammatory values in the blood. PET-CT imaging at three months post intervention did not show signs of infection enclosing the aorta or both femoral regions.

### Discussion

This case demonstrates a successful treatment of a chronically infected occluded aorto-bifemoral Dacron^{®} bypass by a local bacteriophage application. It is assumed that around 50-65% of prosthesis infections are a result of bacterial contamination during surgery [3,4]. A general distinction is made between early (up to 30 days postoperatively) and late infections, although the classification is arbitrary [3,4]. Early prosthesis infections are often assumed to be a consequence of intraoperative contamination and late infections to be a result of hematogenous bacterial spread, but profound evidence for this is limited. Late infections are usually caused by insufficient tissue integration of the prosthesis into the graft bed. Common pathogenic agents are staphylococci, enterobacteria and corynebacteria [3,4]. Bacteriophages (or simply 'phages'; Greek: "bacteria eater") are viruses that selectively infect bacterial cells and were first described in 1917 by the Canadian Félix Hubert d'Hérelle .

Currently Bacteriophages are known as a potent anti-bacterial treatment due to their lytic activity. They are considerably stable when exposed to the inflammatory environment and contribute significantly to the regulation of global bacterial mass. A bacteriophage can only multiply where its host is. They are highly specific and therefore predominantly affect strains within one bacterial species, rarely crossing species boundaries [5].

In the first (lytic) cycle of viral reproduction, phages kill their corresponding bacteria through lysis: once infected, the bacterium host cell then starts the process of reproduction, the destruction of the bacterium, and the release of new phage particles; this process is controlled by enzymes and an interaction of bacterial and phage genes. In the second (lysogenic) cycle, the bacteriophage nucleic acid is integrated into the host bacterium's genome or forms a circular replicon in the bacterial cytoplasm. Compared to other antibacterial therapeutic strategies like local Rifampicine treatment, no cytotoxic effects on vascular cells could be found for bacteriophages.

In addition, they are effective on multi-drug resistant bacteria as well as biofilm-organized bacteria. Recently, in a case series of eight patients with infections of vascular grafts, surgical wounds or implanted medical devices further demonstrated the feasibility of using different bacteriophages with lytic activity for successful treatment of bacterial infections. Although bacteriophages were used for successful treatment of infections of vascular implants, bacteriophage treatment is still not common and not an officially recommended option for infections in the westernized hemisphere. The retro- and intraabdominal application of phages directly to the infection site ensured a maximum concentration, contact time and invasion of the bacteriophages into the infected peri graft tissue. We were able to perform a short operation time, a definite treatment in respect to complete skin/wound closure and the forego of any drainages. No bacteriophage related clinical adverse events had been detected in our case. A three-month follow-up PET- CT scan revealed no signs of infections. It could be assumed that the bacteriophage treatment was successful.

In order to treat the local inflammation in the abdominal and femoral areas in the long term intra- and postoperatively, we perceived the use of bacteriophages as an alternative therapy option in antibacterial local therapy. However, there is an ongoing follow-up for the patient to assure a lasting treatment success. In summary, this case report demonstrates that bacteriophage treatment could be a curative treatment option for patients with bacterial graft- and peri graft infections that are not suitable for extensive surgical approaches.

### References to Example 2:

1. Szilagyi DE, Smith RF, Elliott JP, Vrandecic MP (1972) Infection in arterial reconstruction with synthetic grafts. Ann Surg 176: 321-333.
2. Zühlke HV, Harnoss BM, Lorenz EP (1994) Postoperative Infektionen in der Gefäßchirurgie. In: Septische Gefäßchirurgie Blackwell Wiss Verlag.

### References to Example 3

[3] Kirklin JK, Pagani FD, Kormos RL, Stevenson LW, Blume ED, Myers SL, et al. Eighth annual INTERMACS report: Special focus on framing the impact of adverse events. The Journal of Heart and Lung Transplantation 2017;36:1080-6.
[4] Kim J, Feller ED, Chen W, Liang Y, Dilsizian V. FDG PET/CT for Early Detection and Localization of Left Ventricular Assist Device Infection: Impact on Patient Management and Outcome. JACC Cardiovasc Imaging 2019;12:722-9.
[5] Baddour LM, Wilson WR, Bayer AS, Fowler VG, Tleyjeh IM, Rybak MJ, et al. Infective Endocarditis in Adults: Diagnosis, Antimicrobial Therapy, and Management of Complications: A Scientific Statement for Healthcare Professionals From the American Heart Association. Circulation 2015;132:1435-86.

## Claims

1. A hydrogel comprising hydroxyethyl cellulose (HEC), bacteriophages, CaCl₂ and glycerol.

2. The hydrogel of claim 1, wherein the hydrogel comprises 10% to 33 % CaCl₂, preferably 15% to 25 % CaCl₂.

3. The hydrogel of claim 2, wherein the hydrogel comprises 17% to 23 % CaCl₂, preferably 20% to 22 % CaCl₂, more preferably 21.5 % CaCl₂.

4. The hydrogel of any one of the preceding claims, wherein the hydrogel comprises 3% to 10% HEC.

5. The hydrogel of claim 4, wherein the hydrogel comprises 5 to 8 % HEC, preferably 6% to 7% HEC, more preferably 6,5 % HEC.

6. The hydrogel of any one of the preceding claims, wherein the hydrogel comprises 6% to 22 % glycerol, preferably 8% to 18 % glycerol, more preferably 10% to 16 % glycerol.

7. The hydrogel of claim 6 wherein the hydrogel comprises 12% to 15 % glycerol, preferably 13.5% glycerol.

8. The hydrogel of any one of the preceding claims, wherein the concentration of the bacteriophages is from 10² pfu/ml to 10⁸ pfu/ml.

9. The hydrogel of any one of the preceding claims, further comprising a buffer.

10. The hydrogel of any one of preceding claims, wherein the hydrogel has a viscosity of 1000 mPas up to 100.000 mPas, preferably 10.000 to 80.000 mPas

11. The hydrogel of any one of the preceding claims, wherein the hydrogel is sterile, preferably wherein the hydrogel has been steam sterilized.

12. The hydrogel of any one of the preceding claims for use in a method of treating or preventing a bacterial infection.

13. The hydrogel for use of claim 12, wherein the hydrogel is applied during surgery.

14. The hydrogel for use of claim 13, wherein the hydrogel is used to treat and / or prevent a bacterial infection of an implant.

## Patentansprüche

1. Hydrogel umfassend Hydroxyethylcellulose (HEC), Bacteriophagen, CaCl₂ und Glycerol.

2. Hydrogel nach Anspruch 1, wobei das Hydrogel 10% bis 33 % CaCl₂, vorzugweise 15% bis 25 % CaCl₂, umfasst.

3. Hydrogel nach Anspruch 2, wobei das Hydrogel 17% bis 23 % CaCl₂, vorzugsweise 20% bis 22 % CaCl₂, mehr bevorzugt 21.5 % CaCl₂, umfasst

4. Hydrogel nach einem der vorangehenden Ansprüche, wobei das Hydrogel 3% bis 10% HEC umfasst.

5. Hydrogel nach Anspruch 4, wobei das Hydrogel 5 bis 8 % HEC, vorzugsweise 6% bis 7% HEC, mehr bevorzugt 6,5 % HEC, umfasst.

6. Hydrogel nach einem der vorangehenden Ansprüche, wobei das Hydrogel 6% bis 22 % Glycerol, vorzugsweise 8% bis 18 % Glycerol, mehr bevorzugt 10% bis 16 % Glycerol, umfasst.

7. Hydrogel nach Anspruch 6, wobei das Hydrogel 12% bis 15 % Glycerol, vorzugsweise 13.5 % Glycerol, umfasst.

8. Hydrogel nach einem der vorangehenden Ansprüche, wobei die Konzentration der Bacteriophagen von 10² pfu/ml bis 10⁸ pfu/ml ist.

9. Hydrogel nach einem der vorangehenden Ansprüche, ferner umfassend einen Puffer.

10. Hydrogel nach einem der vorangehenden Ansprüche, wobei das Hydrogel eine Viskosität von 1000 mPas bis zu 100.000 mPas, vorzugsweise 10.000 bis 80.000 mPas, aufweist.

11. Hydrogel nach einem der vorangehenden Ansprüche, wobei das Hydrogel steril ist, vorzugsweise wobei das Hydrogel dampfsterilisiert wurde.

12. Hydrogel nach einem der vorangehenden Ansprüche zur Verwendung in einem Verfahren zur Behandlung oder Prävention einer bakteriellen Infektion.

13. Hydrogel zur Verwendung nach Anspruch 12, wobei das Hydrogel während einer Operation appliziert wird.

14. Hydrogel zur Verwendung nach Anspruch 13, wobei das Hydrogel verwendet wird, um eine bakterielle Infektion eines Implantats zu behandeln und/oder zu verhindern.

## Revendications

1. Un hydrogel comprenant de l'hydroxyéthylcellulose (HEC), des bactériophages, du CaCl₂ et du glycérol.

2. L'hydrogel de la revendication 1, dans lequel l'hydrogel comprend 10 % à 33 % de CaCl₂, de préférence 15 % à 25 % de CaCl2.

3. L'hydrogel de la revendication 2, dans lequel l'hydrogel comprend 17 % à 23 % de CaCl₂, de préférence 20 % à 22 % de CaCl₂, plus préférentiellement 21,5 % de CaCl₂.

4. L'hydrogel de l'une des revendications précédentes, dans lequel l'hydrogel comprend de 3 % à 10 % de HEC.

5. L'hydrogel de la revendication 4, dans lequel l'hydrogel comprend 5 à 8 % de HEC, de préférence 6 à 7 % de HEC, plus préférentiellement 6,5 % de HEC.

6. L'hydrogel de l'une des revendications précédentes, dans lequel l'hydrogel comprend 6 % à 22 % de glycérol, de préférence 8 % à 18 % de glycérol, plus préférentiellement 10 % à 16 % de glycérol.

7. L'hydrogel de la revendication 6, dans lequel l'hydrogel comprend 12 à 15 % de glycérol, de préférence 13,5 % de glycérol.

8. L'hydrogel de l'une des revendications précédentes, dans lequel la concentration des bactériophages est comprise entre 10²pfu/ml et 10⁸pfu/ml.

9. L'hydrogel de l'une des revendications précédentes, comprenant en outre un tampon.

10. L'hydrogel de l'une des revendications précédentes, dans lequel l'hydrogel a une viscosité de 1000 mPas à 100.000 mPas, de préférence de 10.000 à 80.000 mPas.

11. L'hydrogel de l'une des revendications précédentes, dans lequel l'hydrogel est stérile, de préférence dans lequel l'hydrogel a été stérilisé à la vapeur.

12. L'hydrogel de l'une des revendications précédentes pour une utilisation dans une méthode de traitement ou de prévention d'une infection bactérienne.

13. L'hydrogel utilisé selon la revendication 12, dans lequel l'hydrogel est appliqué au cours d'une intervention chirurgicale.

14. L'hydrogel utilisé selon la revendication 13, dans lequel l'hydrogel est utilisé pour traiter et/ou prévenir une infection bactérienne d'un implant.
